# EUROPEAN PATENT SPECIFICATION

(11) **EP 0 966 490 B1**
(45) Date of publication and mention of the grant of the patent: **09.07.2003**
(21) Application number: 98906683.2
(22) Date of filing: 24.02.1998
(51) Int. Cl.: C08F 10/00, C08F 4/70, C07F 9/50, C07F 15/04, B01J 31/24, B01J 31/18

(54) **CATALYST COMPOSITIONS FOR THE POLYMERIZATION OF OLEFINS**
KATALYSATORZUSAMMENSETZUNGEN FÜR DIE POLYMERISATION VON OLEFINEN
COMPOSITIONS CATALYSEURS PERMETTANT LA POLYMERISATION D'OLEFINES

(30) Priority: 13.03.1997 US 40754 P; 18.04.1997 US 44691 P; 01.05.1997 US 45337 P; 02.05.1997 US 45357 P; 02.05.1997 US 45358 P; 06.05.1997 US 45697 P
(43) Date of publication of application: 29.12.1999
(73) Proprietor: EASTMAN CHEMICAL COMPANY, Kingsport, TN 37660 (US)
(72) Inventor: KILLIAN, Christopher, Moore, Gray, TN 37615 (US); MCDEVITT, Jason, Patrick, Wake Forest, NC 27587 (US); MACKENZIE, Peter, Borden, Kingsport, TN 37660 (US); MOODY, Leslie, Shane, Johnson City, TN 37604 (US); PONASIK, James, Allen, Jr., Kingsport, TN 37660 (US)
(74) Representative: Evans, David Charles
(86) International application number: US9803592
(87) International publication number: WO98040420

(56) References cited:
- EP-A- 0 748 821
- WO-A-97/02298
- WO-A-97/47661
- WO-A-98/03521
- WEHMAN P ET AL: "Influence of various P/N and P/P ligands on the palladium -catalysed reductive carbonylation of nitrobenzene" JOURNAL OF ORGANOMETALLIC CHEMISTRY, vol. 535, no. 1-2, 15 May 1997, page 183-193 XP004082178
- SHIRAKAWA E ET AL: "On the Catalytic Cycle of the Palladium-Catalyzed Cross-Coupling Reaction of Alkynylstannane with Aryl Iodide" TETRAHEDRON LETTERS, vol. 38, no. 29, 21 July 1997, page 5177-5180 XP004082975
- JOHNSON L K ET AL: "MEW PD(II)- AND NI(II)-BASED CATALYSTS FOR POLYMERIZATION OF ETHYLENE AND ALPHA-OLEFINS" JOURNAL OF THE AMERICAN CHEMICAL SOCIETY, vol. 117, no. 23, 14 June 1995, page 6414/6415 XP002025459 cited in the application
- VAN DEN BEUKEN, ESTHER K. ET AL: "Oligomerization of ethene by new palladium iminophosphine catalysts" CHEM. COMMUN. (CAMBRIDGE) (1998), (2), 223-224 CODEN: CHCOFS;ISSN: 1359-7345, XP002066973
- JIANG, ZHAOZHONG ET AL: "Stereo- and Enantioselective Alternating Copolymerization of.alpha.-Olefins with Carbon Monoxide. Synthesis of Chiral Polymers" MACROMOLECULES (1994), 27(10), 2694-700 CODEN: MAMOBX;ISSN: 0024-9297, XP000445666
- HVASTIJOVA, M. ET AL: "Structure properties of dicyanamide and tricyanmethanide complexes of copper(II), nickel(II) and cobalt(II) with ligands of pyrazole type - nucleophilic addition in the studied systems" PROC. CONF. COORD. CHEM. (1987), 11TH, 109-14 CODEN: PCCHDB;ISSN: 0139-9535, XP002085229

## Description

### Cross Reference to Related Applications

This application claims the priority from the following United States provisional applications: Provisional Application Serial No. 60/044,691, filed April 18, 1997; Provisional Application Serial No. 60/045,337, filed May 1, 1997; Provisional Application Serial No. 60/045,358, filed May 2, 1997; Provisional Application Serial No. 60/045,357, filed May 2, 1997; and Provisional Application Serial No. 60/045,697, filed May 6, 1997.

### Field of the Invention

The present invention relates to catalyst compositions for olefin polymerization and processes for preparing polyolefins utilizing the catalysts. More particularly, the present invention relates to catalyst compositions comprising ligand complexes comprising nitrogen or nitrogen and phosphorus, for example, phosphine groups, heterocycle groups or imine groups.

### Background of the Invention

Olefin polymers are used in a wide variety of products, from sheathing for wire and cable to film. Olefin polymers are used, for instance, in injection or compression molding applications, in extruded films or sheeting, as extrusion coatings on paper, for example photographic paper and digital recording paper, and the like. Improvements in catalysts have made it possible to better control polymerization processes, and, thus, influence the properties of the bulk material. Increasingly, efforts are being made to tune the physical properties of plastics for lightness, strength, resistance to corrosion, permeability, optical properties, and the like, for particular uses. Chain length, polymer branching and functionality have a significant impact on the physical properties of the polymer. Accordingly, novel catalysts are constantly sought in attempts to obtain a catalytic process for polymerizing olefins which permits more efficient and better controlled polymerization of olefins.

Conventional polyolefins are prepared by a variety of polymerization techniques, including liquid phase, heterogeneous gas phase, and slurry polymerizations. Certain transition metal catalysts, such as those based on titanium compounds (e.g. TiCl₃ or TiCl₄) in combination with organoaluminum cocatalysts, are used to make linear and linear low density polyethylenes as well as poly-α-olefins such as polypropylene. These so-called "Ziegler-Natta" catalysts are quite sensitive to oxygen and are ineffective for the copolymerization of nonpolar and polar monomers.

Recent advances in non-Ziegler-Natta olefin polymerization catalysis include the following:
L. K. Johnson *et al*., WO Patent Application 96/23010, discloses the polymerization of olefins using cationic nickel, palladium, iron, and cobalt complexes containing diimine and bisoxazoline ligands. This document also describes the polymerization of ethylene, acyclic olefins, and/or selected cyclic olefins and optionally selected unsaturated acids or esters such as acrylic acid or alkyl acrylates to provide olefin homopolymers or copolymers;
European Patent Application Serial No. 381,495, describes the polymerization of olefins using palladium and nickel catalysts which contain selected bidentate phosphorous containing ligands;
L. K. Johnson *et al., J. Am. Chem. Soc*., **1995,** *117*, 6414, describes the polymerization of olefins such as ethylene, propylene, and 1-hexene using cationic α-diimine-based nickel and palladium complexes. These catalysts have been described to polymerize ethylene to high molecular weight branched polyethylene. In addition, Pd complexes act as catalysts for the polymerization and copolymerization of olefins and methyl acrylate (L. K. Johnson *et al*., *J. Am. Chem. Soc.,* **1996,** *118,* 267).
G.F. Schmidt *et al., J. Am. Chem. Soc*. **1985,** *107*, 1443, describes a cobalt(III) cyclopentadienyl catalytic system having the structure [C₅Me₅(L)CoCH₂CH₂-µ-H]⁺, which provides for the "living" polymerization of ethylene. In addition, M. Brookhart et al., *Macromolecules* **1995,** *28*, 5378, discloses using such "living" catalysts in the synthesis of end-functionalized polyethylene homopolymers;
U. Klabunde, U. S. Patent Nos. 4,906,754; 4,716,205; 5,030,606; 5,175,326; describes the conversion of ethylene to polyethylene using anionic phosphorous/oxygen donors ligated to Ni(II). The polymerization reactions were run between 25 and 100 °C with modest yields, preparing linear polyethylene having a weight-average molecular weight ranging between 8K and 350K. In addition, Klabunde describes the preparation of copolymers of ethylene and functional group containing monomers;
M. Peuckert *et al*., *Organomet*. **1983,** *2*(5), 594, discloses the oligomerization of ethylene using phosphine/carboxylate donors ligated to Ni(II), which demonstrated modest catalytic activity (0.14 to 1.83 TO/s). The oligomerizations were carried out at 60 to 95 °C and 10 to 80 bar ethylene in toluene, to produce linear α-olefins;
R.E. Murray, U.S. Patents Nos. 4,689,437 and 4,716,138, describes the oligomerization of ethylene using phosphine/sulfonate donors ligated to Ni(II). These complexes show catalyst activities approximately 15 times greater than those reported with phosphine/carboxylate analogs;
W. Keim *et al., Angew. Chem. Inf. Ed. Eng*. **1981,** *20*, 116, and V.M. Mohring, *et al., Angew. Chem. Int. Ed. Eng*. **1985,** *24*, 1001, disclose the polymerization of ethylene and the oligomerization of α-olefins with aminobis(imino)phosphorane nickel catalysts; G. Wilke, *Angew. Chem. Int*. *Ed. Engl*. **1988,** *27*, 185, describes a nickel allyl phosphine complex for the polymerization of ethylene.
K.A.O. Starzewski *et al., Angew. Chem. Int. Ed*. *Engl*. **1987,** *26,* 63 and U. S. Patent 4,691,036, describes a series of bis(ylide)nickel complexes, used to polymerize ethylene to provide high molecular weight linear polyethylene;
WO Patent Application 97/02298, discloses the polymerization of olefins using a variety of neutral N, O, P, or S donor ligands, in combination with a nickel(0) compound and an acid;
Brown *et al.,* WO 97/17380, describes the use of Pd α-diimine catalysts for the polymerization of olefins including ethylene in the presence of air and moisture;
Fink *et al.,* U. S. Patent No. 4,724,273, describes the polymerization of α-olefins using aminobis(imino)phosphorane nickel catalysts and the compositions of the resulting poly(α-olefins);
Additional recent developments are described by Sugimura *et al*., in JP96-84344, JP96-84343, and WO 9738024, and by Yorisue *et al*., in JP96-70332

Notwithstanding these advances in non-Ziegler-Natta catalysis, there remains a need for efficient and effective Group 8-10 transition metal catalysts for effecting polymerization of olefins. In addition, there is a need for novel methods of polymerizing olefins employing such effective Group 8-10 transition metal catalysts. In particular, there remains a need for Group 8-10 transition metal olefin polymerization catalysts with both improved temperature stability and functional group compatibility. Further, there remains a need for a method of polymerizing olefins utilizing effective Group 8-10 transition metal catalysts in combination with a Lewis acid so as to obtain a catalyst that is more active and more selective.

### Summary of the Invention

The present invention includes novel ligands, which may be utilized as part of a catalyst system. A catalyst system of the present invention is a transition metal - ligand complex. In particular, the catalyst system is comprised of a transition metal component and a ligand component comprising a nitrogen atom, generally in the form of an imine or heterocycle group. In certain embodiments, the ligand component may further comprise a phosphorous atom. Preferred ligand components are bidentate and include a nitrogen- transition metal bond. The transition metal - ligand complex is generally cationic and associated with a weakly coordinating anion.

A catalyst system of the present invention may further comprise a Lewis or Bronsted acid. The Lewis or Bronsted acid may be complexed with the transition metal component and/or the ligand component of the transition metal-ligand complex.

In one aspect of the present invention, the transition metal component of the catalyst system is Group 8-10 transition metals. In another aspect, the catalyst system further comprises a Lewis or Bronsted acid and the transition metal component is Group 8-10 transition metals. Preferred transition metal components include iron (Fe), cobalt (Co), nickel (Ni) and palladium (Pd). The choice of a particular transition metal may be made in view of the end use of the catalyst system.

The present invention also provides processes for preparing polyolefins, preferably having a degree of polymerization of at least 10, comprising contacting one or more monomers, preferably selected from the group comprising R'CH=CHR" wherein R' and R" are independently hydrogen, hydrocarbyl, fluoralkyl, or R' and R" collectively form a cyclic olefin, with a catalyst system of the present invention at a temperature and a pressure sufficient to effect polymerization, preferably a temperature of -100 to 200 °C, and a pressure of from about 1 atmosphere to 100 atmospheres.

The catalyst system of this invention is extremely versatile in that changes in the ligand or changes to the transition metal itself can be made to obtain a "tailor made" catalyst to suit a particular set of requirements for a particular monomer and polymer. In certain embodiments of the present invention, a Lewis or Bronsted acid may be used as a co-catalyst to render the transition metal more electron deficient, and therefore more active and/or selective, and/or act as a site to bind the catalyst to a surface.

Further features and advantages of the catalyst system and processes of the present invention will become more apparent from the following more detailed description.

### Detailed Description of the Invention

The novel ligand components of the present invention are broadly described as comprising a nitrogen atom, preferably in the form of an imine or heterocyclic functional group.

A catalyst system of the present invention may further comprise a Lewis or Bronsted acid, which may be complexed with the ligand component or the transition metal component. While not wishing to be bound by theory, it is believed that the Lewis acid complexation can render the transition metal more electron deficient, and therefore more active and/or selective, and/or act as a site to bind the catalyst to a surface. Although this strategy may be implemented in a variety of ways, ligands in which the Lewis acid is bound to one or more heteroatoms which are π-conjugated to the donor atom or atoms bound to the transition metal are preferred.

In one aspect of the present invention, the transition metal component of the catalyst system is a Group 8-10 transition metal. In another aspect, the catalyst system further is a Lewis or Bronsted acid and the transition metal component is a Group 8-10 transition metal. A catalyst system of the present invention may advantageously be utilized in a process for the polymerization of olefins, including ethylene and α-olefins such as propylene and 1-hexane and cyclic olefins such as cyclopentene and norbornene. Accordingly, a process of the present invention is contacting one or more monomers comprising R'CH=CHR" with a catalyst system of the present invention at a temperature and a pressure sufficient to effect polymerization, preferably a temperature of -100 to 200 °C, more preferably a temperature from 25 to 150 °C, and a pressure of from about 1 atmosphere to 100 atmospheres, wherein R' and R" are independently hydrogen, hydrocarbyl, fluoroalkyl, or R' and R" may be linked to form a cyclic olefin. The possible embodiments of a process of the present invention for the production of polyolefins include processes utilizing the catalyst systems of the present invention described herein.

The embodiments of a catalyst system of the present invention are described in detail below utilizing the following terms defined as follows:

Symbols ordinarily used to denote elements in the Periodic Table take their ordinary meaning, unless otherwise specified. Thus, N, O, S, P, and Si stand for nitrogen, oxygen, sulfur, phosphorus and silicon, respectively.

Examples of neutral Lewis bases include, but are not limited to, ethers, organic nitriles or organic sulfides.

Examples of Lewis acids include, but are not limited to, methylalumoxane (hereinafter MAO) and other aluminum sesquioxides, R⁷₃Al, R⁷₂AlCl, R⁷AlCl₂ (where R⁷ is alkyl), organoboron compounds, boron halides, B(C₆F₅)₃, R⁹₃Sn[BF₄], (where R⁹ is alkyl or aryl), MgCl₂, and H⁺X⁻, where X⁻ is a weakly coordinating anion.

A "hydrocarbyl" group means a monovalent or divalent, linear, branched or cyclic group which contains only carbon and hydrogen atoms. Examples of monovalent hydrocarbyls include the following: C₁-C₂₀ alkyl; C₁-C₂₀ alkyl substituted with one or more groups selected from C₁-C₂₀ alkyl, C₃-C₈ cycloalkyl or aryl; C₃-C₈ cycloalkyl; C₃-C₈ cycloalkyl substituted with one or more groups selected from C₁-C₂₀ alkyl, C₃-C₈ cycloalkyl or aryl; C₆-C₁₄ aryl; and C₆-C₁₄ aryl substituted with one or more groups selected from C₁-C₂₀ alkyl, C₃-C₈ cycloalkyl or aryl. As used herein, the term "aryl" preferably denotes a phenyl, napthyl, or anthracenyl group. When the above groups are substituted, they are preferably substituted from one to four times with the listed groups. Examples of divalent (bridging hydrocarbyls) include: -CH₂-, -CH₂CH₂-, -C₆H₄-, and -CH₂CH₂CH₂-.

A "silyl" group refers to a SiR₃ group where Si is silicon and R is hydrocarbyl or substituted hydrocarbyl or silyl, as in Si(SiR₃)_{3.}

A "heteroatom" refers to an atom other than carbon or hydrogen. Preferred heteroatoms include oxygen, nitrogen, phosphorus, sulfur, selenium, arsenic, chlorine, bromine, and fluorine.

The term "fluoroalkyl" as used herein refers to a C₁-C₂₀ alkyl group substituted with one or more fluorine atoms.

A "substituted hydrocarbyl" refers to a monovalent or divalent hydrocarbyl substituted with one or more heteroatoms. Examples of monovalent substituted hydrocarbyls include: trifluoromethyl, 2,6-dimethyl-4-methoxyphenyl, 2,6-diisopropyl-4-methoxyphenyl, 4-cyano-2,6-dimethylphenyl, 2,6-dimethyl-4-nitrophenyl, 2,6-difluorophenyl, 2,6-dibromophenyl, 2,6-dichlorophenyl, 4-methoxycarbonyl-2,6-dimethylphenyl, 2-tert-butyl-6-chlorophenyl, 2,6-dimethyl-4-phenylsulfonylphenyl, 2,6-dimethyl-4-trifluoromethylphenyl, 2,6-dimethyl-4-trimethylammoniumphenyl (associated with a weakly coordinating anion), 2,6-dimethyl-4-hydroxyphenyl, 9-hydroxyanthr-10-yl, 2-chloronapth-1-yl, 4-methoxyphenyl, 4-nitrophenyl, and 9-nitroanthr-10-yl. Examples of divalent substituted hydrocarbyl include: 4-methoxy-1,2-phenylene, 1-methoxymethyl-1,2-ethanediyl, 1,2-bis(benzyloxymethyl)-1,2-ethanediyl, and 1-(4-methoxyphenyl)-1,2-ethanediyl.

A "mono-olefin" means a hydrocarbyl group having one carbon-carbon double bond.

A "suitable nickel precursor" refers to a nickel compound which may be combined with compound **III, VI, IX,** or **XII,** and optionally a Lewis or Bronsted acid, to form an active olefin polymerization catalyst. Examples include: bis(1,5-cycloctadiene)nickel(0), (1,2-dimethoxyethane)nickel(II) dibromide, bis[(chloro)(1, 2, 3-η³-2-propenyl) nickel(II)].

The term "polymer" as used herein is meant a species comprised of monomer units and having a degree of polymerization (DP) of ten or higher.

The term "weakly coordinating counterion" is well-known in the art *per se* and generally refers to a large bulky anion capable of delocalization of the negative charge of the anion. Suitable weakly coordinating anions include, but are not limited to, PF₆⁻, BF₄⁻, SbF₆⁻, (Ph)₄B⁻ where Ph = phenyl, ⁻BAr₄ where ⁻BAr₄ = tetrakis[3,5-bis(trifluoromethyl)phenyl]borate. The coordinating ability of such anions is known and described in the literature (Strauss, S. *et al., Chem. Rev.* **1993,** *93*, 927).

As used herein, the terms "monomer" or "olefin monomer" refer to the olefin or other monomer compound before it has been polymerized. The term "monomer units" refers to the moieties of a polymer that correspond to the monomers after they have been polymerized;

In some cases, a compound Y is required as a cocatalyst. Suitable compounds Y include a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion. Preferred compounds Y include: methylalumoxane (hereinafter MAO) and other aluminum sesquioxides, R⁷₃Al, R⁷₂AlCl, R⁷AlCl₂ (where R⁷ is alkyl), organoboron compounds, boron halides, B(C₆F₅)₃, R⁹₃Sn[BF₄], (where R⁹ is alkyl or aryl), MgCl₂, and H⁺X⁻, where X⁻ is a weakly coordinating anion.

Especially preferred olefin monomers include ethylene, propylene, 1-butene, 1-pentene, 1-hexene, 1-heptene, cyclopentene, and norbomene.

The present invention provide catalyst systems comprising transition metal-ligand complexes wherein the ligand component is a bidentate ligand which binds to a transition metal and is a heterocycle and an additional nitrogen donor group such as an imine.

The present invention provides a catalyst system comprising a transition metal complex of bidentate ligands (imino-substituted heterocycles) having a five-membered ring, formed by the metal complex, and comprising one metal atom, one carbon atom, and three nitrogen atoms.

The olefin polymerization catalysts of the present invention comprise transition metal complexes of bidentate ligands that can be referred to as imino-substituted heterocycles, more specifically imino-substituted pyrazoles and other related heterocycles with adjacent 1,2-nitrogen atoms. The transition metal component is a Group 8-10 transition metal. Nickel, cobalt and palladium are preferred transition metals. The ligands may also be referred to using a "imine/heterocycle" or "heterocycle/ imine" nomenclature which describes the two components of the bidentate ligand, e.g., imine/triazole. The five-membered ring formed by the transition metal complex contains one transition metal atom, one carbon atom, and three nitrogen atoms (two of which are provided by the heterocycle component of the bidentate ligand).

One embodiment of the present invention involves a catalyst of the formula **XVI:** 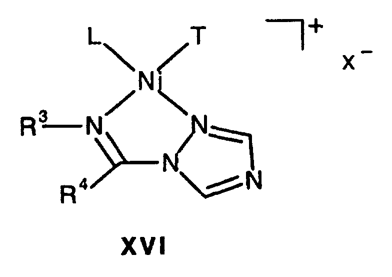 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

Another embodiment of the present invention involves a catalyst of the formula XVII: 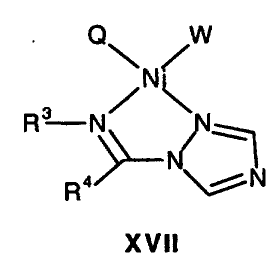 wherein:
R³ is hydrocarbyl or substituted hydrocarbyl;
R⁴ is hydrogen, hydrocarbyl, substituted hydrocarbyl, or silyl;
Q is a hydrocarbyl, chloride, iodide, or bromide; and
W is a hydrocarbyl, chloride, iodide, or bromide.

The invention also provides processes for preparing polyolefins utilizing the compounds of formula **XVI** and **XVII**.

Thus, the present invention provides a process for preparing polyolefins comprising contacting one or more olefin monomers having the composition R'CH=CHR" wherein R' and R" are independently hydrogen, hydrocarbyl, fluoroalkyl, or R' and R" may be linked to form a cyclic olefin; with a catalyst comprising the formula **XVI**: 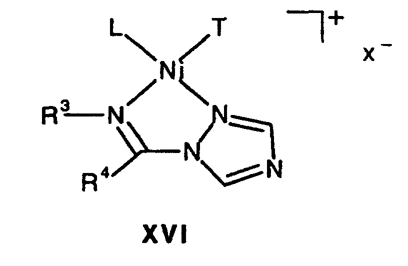 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion at a temperature and a pressure sufficient to effect polymerization.

Accordingly, the invention also provides a process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with the catalyst system comprising a compound of the formula **XVII** and a second compound Y: 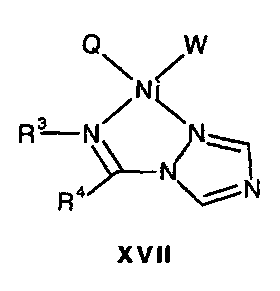 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide; and
W is hydrocarbyl, chloride, iodide, or bromide;
and Y is a neutral Lewis acid capable of abstracting Q⁻ or W⁻ to form a weakly coordinating anion, a cationic Lewis acid whose counterion is a weakly coordinating anion, or a Bronsted acid whose conjugate base is a weakly coordinating anion; at a temperature and a pressure sufficient to effect polymerization.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

Examples of processes for preparing polyolefins utilizing complex **IV** or **V** of the second embodiment of the present invention are set forth in the following examples.

### Further Embodiment of the Present Invention

In a further embodiment, the present invention provides a catalyst system comprising a transition metal - ligand complex further complexed with one or more Lewis acids to obtain an active olefin polymerization catalyst. Suitable ligands for use in the transition metal-ligand complex comprise the ligand components described above. A preferred transition metal is nickel.

Examples include methylaluminoxane, alkylaluminums, alkylaluminum halides, organoboron compounds, boron halides, B(C₆F₅)₃, Li[BF₄], R₃Sn[BF₄], MgCl₂, M₃SiCl/SnCl₄, SbCl₅, alumina H⁺X⁻, where X⁻ is a relatively non-coordinating anion, and Montmorillonite clay.

An electron deficient metal center is advantageous for efficient olefin polymerization. This embodiment of the present invention provides catalyst systems wherein a binucleating or multinucleating ligand is complexed to a transition metal and one or more Lewis acids to obtain an active olefin polymerization catalyst. While not wishing to be bound by any theory, it is believed that the Lewis acid complexation renders the transition metal more electron deficient rendering the transition metal more active and/or selective, and/or available as a site to bind the catalyst system to a surface. Thus, broadly, catalyst systems of this embodiment of the present invention comprise ligand components, such as the ligand components described in the foregoing, complexed with a transition metal and a Lewis acid. Preferred catalyst systems of this embodiment of the present invention include catalyst systems wherein the Lewis acid is bound to one or more heteroatoms which are π-conjugated to the donor atom or atoms bound to the transition metal.

Examples of catalyst systems comprising a Lewis acid - ligand - transition metal complex of the fifth embodiment of the present invention include **XIX**: 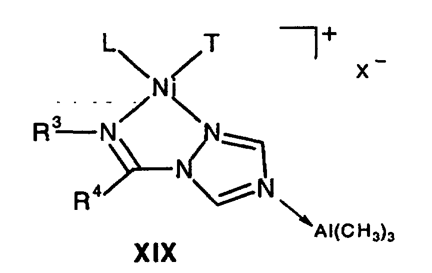 wherein
R3, R4, L, and T are defined as above.

Complex **XIX** may be produced by synthesizing the transition metal - ligand complex in the manner described above and exemplified in the example herein, and then combining the transition metal - ligand complex with the Lewis acid component.

A process of the present invention utilizing this embodiment may be described as follows.

A process for preparing polyolefins comprising contacting one or more olefin monomers with a catalyst system at a temperature and a pressure sufficient to effect polymerization, wherein the catalyst system comprises a transition metat-ligand-Lewis acid complex, e.g. complex XIX of this embodiment of the present invention.

Preferred polyolefin products will have a degree of polymerization (DP) of at least 10. A preferred olefin monomer is R'CH=CHR" wherein R' and R" are independently hydrogen, hydrocarbyl, fluoroalkyl, or R' and R" may be linked to form a cyclic olefin. Preferred temperature conditions are -100 to 200 °C. Preferred pressure conditions are from about 1 atmosphere to 100 atmospheres.

A preferred embodiment of the present invention involves a composition comprising (i) a Group 8-10 transition metal M, (ii) one or more Lewis acids, and (iii) a binucleating or multinucleating compound of the formula **XVIII**: 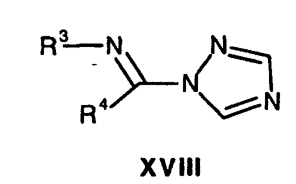 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl.

The features and advantages of the present invention are further illustrated by the following examples. The procedures described in each example were conducted utilizing conventional laboratory equipment known to those of ordinary skill in the art.

### Ligand Synthesis

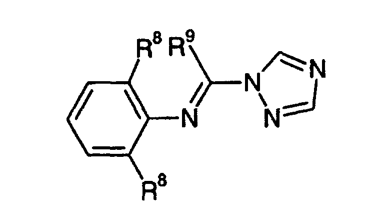

**C1:** R⁸ = Me, R⁹ = 1-naphthyl
**C2:** R⁸ = iPr, R⁹ = 1-naphthyl
**C3:** R⁸ = Me, R⁹ = phenyl
**C4:** R⁸ = iPr, R⁹ = phenyl
**C5:** R⁸ = iPr, R⁹ = 2,6-dimethoxyphenyl

### Example 1

Synthesis of **C1.** Equimolar amounts of 1,2,4-triazole and *N*-(2,6-dimethylphenyl)-1-naphthimidoyl chloride (derived in the conventional fashion [H. Ulrich, *The Chemistry of Imidoyl Halides,* Plenum, New York, 1968] from the corresponding amide and phosphorus pentachloride) were treated with an excess of triethylamine in methylene chloride at room temperature. After 1 hour, the solution was concentrated, taken up in ethyl acetate, filtered, and washed sequentially with saturated sodium bicarbonate solution, 0.5M HCI solution, and brine, then dried over magnesium sulfate, filtered, and concentrated to a syrup. Purification by flash chromatography (hexane/EtOAc = 4:1) afforded the triazole/imine ligand **C1** as a pale yellow solid.

### Example 2

Synthesis of **C2.** A mixture of 1,2,4-triazole (1.2 mmol) and *N*-(2,6-diisopropylphenyl)-1-naphthimidoyl chloride (0.5 mmol) was treated with triethylamine (1 mmol) and methylene chloride (5 mL) and stirred four hours at room temperature. The reaction was worked up as described in example 20 to afford **C2** as a pale yellow solid.

### Example 3

Synthesis of **C3.** A mixture of 1,2,4-triazole (2.9 mmol) and N-(2,6-dimethylphenyl)-1-benzimidoyl chloride (1.5 mmol) was treated with triethylamine (1.5 mmol) and methylene chloride (7 mL) and stirred 14 hours at room temperature. The reaction was worked up as described in example 20 to afford **C3** as a pale yellow solid (242 mg).

### Example 4

Synthesis of **C4.** A mixture of 1,2,4-triazole (1.5 mmol) and N-(2,6-diisopropylphenyl)-1-benzimidoyl chloride (0.7 mmol) was treated with triethylamine (1 mmol) and methylene chloride (6 mL) and stirred overnight at room temperature. The reaction was worked up as described in example 20 to afford C4 as a pale yellow solid (85 mg) after purification by flash chromatography (hexane/EtOAc =3:1).

### Example 5

Synthesis of **C5.** 2,6-Diisopropylaniline (25 mmol) was added to a chilled suspension of 2,6-dimethoxybenzoyl chloride (25 mmol) in pyridine (20 mL). After stirring three hours, water (50 mL) was added, preparing a purple precipitate that was isolated by filtration and subsequently recrystallized from ethanol to provide the amide as a purple solid.

A solution of the amide (690 mg, 2 mmol) in acetonitrile (8 mL) was added to a mixture formed by the addition of triethylamine (580 µL) to a cooled suspension of 1,2,4-triazole (420 mg, 6 mmol) and POCl₃ in acetonitrile (8 mL). After stirring 24 hours at room temperature, the reaction contents were filtered, and the filtrate was evaporated to an oil. The oil was dissolved in EtOAc, and washed successively with aqueous solutions of sodium bicarbonate, HCI, and NaCI. The organic layer was dried over magnesium sulfate, filtered, and concentrated to an oil. Purification by flash chromatography (hexane/EtOAc = 3:1) afforded the desired imino-substituted heterocyclic **C5.**
**D1:** R⁸ = Me, R⁹ = 1-naphthyl
**D2:** R⁸ = iPr, R⁹ = 1-naphthyl
**D3:** R⁸ = Me, R⁹ = phenyl
**D4:** R⁸ = iPr, R⁹ = phenyl
**D5:** R⁸ = iPr, R⁹ = 2,6-dimethoxyphenyl

### Example 6

Synthesis of **D1.** A methylene chloride solution of the triazole/imine **C1** (46 mg, 0.14 mmol) and (DME)NiBr₂ (37 mg, 0.12 mmol) were combined via stainless steel cannula. The mixture was left to stir at 23 °C for 16 hours. The solvent was partially evaporated under reduced pressure and 10 mL of hexane was added to fully precipitate the complex. The resulting yellow solid was washed with hexane and then placed under high vacuum to remove all solvent. 37 mg of pre-catalyst **D1** isolated (60 % yield).

### Example 7

Synthesis of **D2.** A flame dried Schlenk flask equipped with a magnetic stir bar and capped with a rubber septum. To the flask was added DMENiBr₂ (59 mg, 0.191 mmol) and 10 mL of methylene chloride. In a separate flask the imine/triazole **C2** (78 mg, 0.204mmol) was dissolved in 5 mL of methylene chloride and transferred via stainless steel canula onto the DMENiBr₂ suspension. The mixture was stirred at room temperature for 16 hours. After 16 hours, the methylene chloride was removed *in vacuo* resulting in a yellow/green solid. The solid was washed with 2 X 10 mL of hexane. The solid was left to dry under reduced pressure for several hours resulting in 42 mg of the imine/triazole complex **D2.**

### Example 8

Synthesis of **D3.** Methylene chloride (8 mL) was added to a mixture of triazole/imine **C3** (54 mg) and (DME)NiBr₂ (57 mg). The reaction was stirred at room temperature overnight. Solvent was removed via filter cannula, and the pale yellow powder was washed with hexane, then dried *in vacuo*.

### Example 9

Synthesis of **D4.** Methylene chloride (6 mL) was added to a mixture of triazole/imine **C4** (60 mg) and (DME)NiBr₂ (45 mg). The reaction was stirred at room temperature overnight. The solvent was partially evaporated under a stream of argon, and hexane was added. The precipitated pale green complex was washed several times with hexane, then dried *in vacuo*.

### Example 10

Synthesis of **D5.** Methylene chloride (9 mL) was added to a mixture of ligand **C5** (125 mg) and (DME)NiBr₂ (83 mg). The reaction was stirred at room temperature overnight. The solvent was partially evaporated under a stream of argon, and hexane was added. The precipitated pale green complex was washed several times with hexane, then dried *in vacuo*.

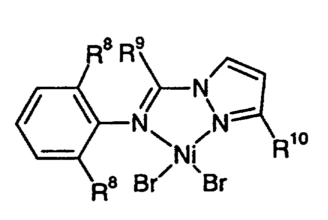

### POLYMERIZATIONS

### Example 11

The triazole/imine complex **D1** (2.0 mg) was suspended in 50 mL of dry toluene. The reaction mixture was equilibrated at room temperature under an ethylene atmosphere, then treated with MAO (2.0 mL of a 10 % by weight solution in toluene) and stirred under 1 atm ethylene. A white polyethylene precipitate was observed within minutes. After five minutes, the reaction was quenched by the sequential addition of acetone, methanol, and 6M HCI. The precipitate was isolated by filtration, washed, and dried *in vacuo* to yield 700 mg of polyethylene (85,000 To/h). DSC: Tₘ = 123. GPC: M_{w} =23.000.

### Example 12

The triazole/imine complex **D3** (2.7 mg) was suspended in 20 mL of dry toluene. The reaction mixture was equilibrated at 0°C under an ethylene atmosphere, then treated with MAO (2.0 mL of a 10 % by weight solution in toluene) and stirred under 1 atm ethylene. After 15 minutes, the reaction was quenched by the sequential addition of acetone, methanol, and 6M HCI. The precipitate was isolated by filtration, washed, and dried *in vacuo* to yield 747 mg of polyethylene. (19,600 To/h, Mn = 6900, Mw = 52,200 (GPC); 2 branches/1000 carbons by NMR)

### Example 13

A flame dried Schlenk flask was charged with complex **D1** (5 mg, 0.0092 mmol), norbomene (2g) and 50 mL of toluene. The flask was then placed in a water bath (23 C) to control reaction temperatue. MAO (2 mL of a 10 wt.% solution in toluene) was then added to the suspension and the reaction left to stir for 16 hours. Acetone and methanol were added to quench the reaction and precipitate the resulting polynorbomene. The polymer was collected by filtration and washed with 6M HCl, water, and acetone. The polymer was dried in a vacuum oven resulting in 700 mg of polynorbomene. GPC: Mₙ = 14,500; M_{w} = 44,000.

### Example 14

A flame dried Schlenk flask was charged with complex **D4** (6 mg, 0.0091 mmol), 1 atmosphere ethylene and 50 mL of toluene. The flask was then placed in a water bath (23 C) to control reaction temperatue. MAO (2 mL of a 10 wt.% solution in toluene) was then added to the suspension and the reaction left to stir for 20 minutes (polymer began to precipitate within minutes). Acetone, methanoland 6M HCl were added to quench the reaction and precipitate the resulting polyethylene. The polymer was dried in a vacuum oven resulting in 400 mg of polyethylene. GPC: Mₙ = 2100; M_{w} = 5600. ¹H NMR: 30 branches/1000 carbon atoms. DSC: Tₘ = 104 C.

### Example 15

A flame dried Fisher-Porter bottle was charged with complex **D2** (5 mg, 0.0083 mmol) and 50 mL of toluene. The flask was placed in a water bath (23 C) to control reaction temperatue. MAO (2 mL of a 10 wt.% solution in toluene) was then added to the suspension and the bottle rapidly pressurized to 45 psig and the reaction left to stir for 20 minutes. Acetone, methanol and 6M HCl were added to quench the reaction and precipitate the resulting polyethylene. The polymer was dried in a vacuum oven resulting in 150 mg of polyethylene. GPC: M_{w} = 22,000. ¹H NMR: 7 branches/1000 carbon atoms. DSC: Tₘ = 127 C.

### Example 16

A flame dried Schlenk flask was charged with complex **D4** (2 mg, 0.0036 mmol), 1 atmosphere ethylene and 50 mL of toluene. The flask was then placed in an ice-water bath (0 C) to control reaction temperatue. MAO (2 mL of a 10 wt.% solution in toluene) was then added to the suspension and the reaction left to stir for 10 minutes (polymer began to precipitate within minutes). Acetone, methanol and 6M HCI were added to quench the reaction and precipitate the resulting polyethylene. The polymer was dried in a vacuum oven resulting in 560 mg of polyethylene (33,000 TO/h). GPC: Mₙ = 5500; M_{w} = 27,000. ¹H NMR: 4 branches/1000 carbon atoms. DSC: Tₘ = 130 C.

### Example 17

A flame dried Schlenk flask was charged with complex D4 (2 mg, 0.0036 mmol), 1 atmosphere ethylene and 50 mL of toluene. The flask was then placed in a water bath (23 C) to control reaction temperatue. MAO (2 mL of a 10 wt.% solution in toluene) was then added to the suspension and the reaction left to stir for 10 minutes (polymer began to precipitate within minutes). Acetone, methanol and 6M HCl were added to quench the reaction and precipitate the resulting polyethylene. The polymer was dried in a vacuum oven resulting in 490 mg of polyethylene (30,000 TO/h). GPC: Mₙ = 1600; M_{w} = 6100. ¹H NMR: 20 branches/1000 carbon atoms. DSC: Tₘ = 110 C.

## Claims

1. A catalyst comprising the formula **XVI**: 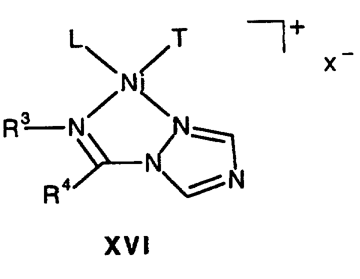 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

2. A process for preparing polyolefins comprising contacting one or more olefin monomers having the composition R'CH=CHR" wherein R' and R" are independently hydrogen, hydrocarbyl, fluoroalkyl, or R' and R" may be linked to form a cyclic olefin; with a catalyst comprising the formula **XVI:** 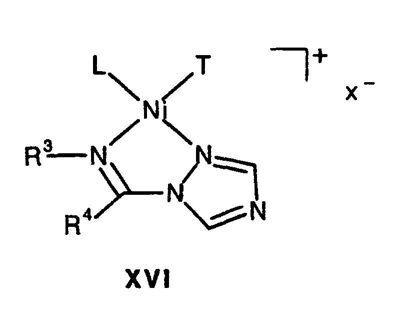 wherein:
R³ is hydrocarbyl or substituted hydrocarbyl;
R⁴ is hydrogen, hydrocarbyl, substituted hydrocarbyl, or silyl;
T is hydrogen, hydrocarbyl, or substituted hydrocarbyl;
L is a monoolefin or a neutral Lewis base where the donating atom is nitrogen, oxygen or sulfur; and
X⁻ is a weakly coordinating anion.

3. A catalyst system comprising a compound of the formula **XVII**: 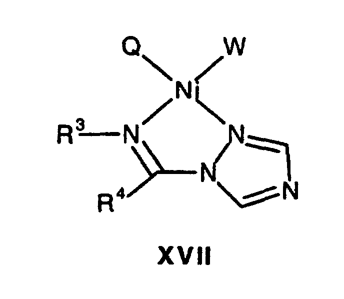 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide;
W is hydrocarbyl, chloride, iodide, or bromide.

4. A process for preparing polyolefins comprising contacting one or more olefin monomers of the formula R'CH=CHR" wherein R' and R" are independently hydrogen, hydrocarbyl, fluoroalkyl, or R' and R" may be linked to form a cyclic olefin; with a catalyst system comprising a compound of the formula **XVII:** 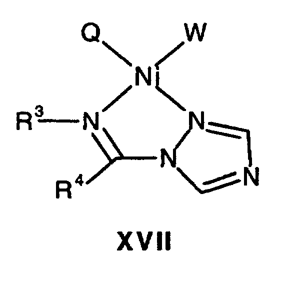 wherein:
R³ is hydrocarbyl or substituted hydrocarbyl;
R⁴ is hydrogen, hydrocarbyl, substituted hydrocarbyl, or silyl;
Q is hydrocarbyl, chloride, iodide, or bromide; and
W is hydrocarbyl, chloride, iodide, or bromide.

5. A composition comprising (i) a Group 8-10 transition metal M, (ii) one or more Lewis acids, and (iii) a binucleating or multinucleating compound of the formula **XVIII**: 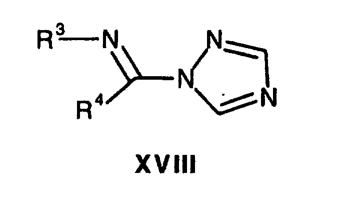 wherein the Lewis acid or acids are bound to one or more heteroatoms which are π-conjugated to the donor atom or atoms bound to the transition metal M;
R³ is hydrocarbyl or substituted hydrocarbyl;
R⁴ is hydrogen, hydrocarbyl, substituted hydrocarbyl, or silyl.

6. The composition of Claim 5 wherein the transition metal M is Ni(II), and the Lewis acid is a boron or aluminum containing Lewis acid.

## Patentansprüche

1. Katalysator, umfassend die Formel **XVI**: 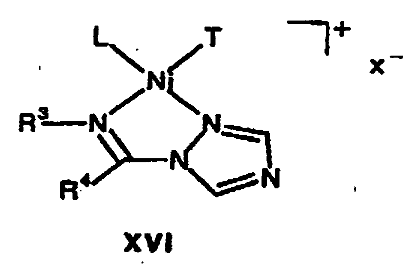 in der
R³ Kohlenwasserstoff oder substituierter Kohlenwasserstoff ist,
R⁴ Wasserstoff, Kohlenwasserstoff, substituierter Kohlenwasserstoff, oder Silyl ist,
T Wasserstoff, Kohlenwasserstoff, substituierter Kohlenwasserstoff ist,
L ein Monoolefin oder eine neutrale Lewis-Base ist, wobei das Donor-Atom Stickstoff, Sauerstoff oder Schwefel ist, und
X⁻ ein schwach koordinierendes Anion ist.

2. Verfahren zur Herstellung von Polyolefinen, umfassend das Zusammenbringen von einem oder mehreren Olefin-Monomeren der Formel R'CH=CHR", in der R' und R" unabhängig Wasserstoff, Kohlenwasserstoff, Fluoralkyl sind, oder R' und R" unter Bildung eines cyclischen Olefins verbunden sein können, mit einem Katalysator umfassend die Formel XVI: 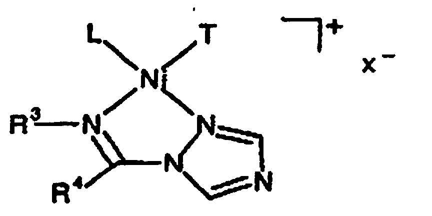 in der
R³ Kohlenwasserstoff oder substituierter Kohlenwasserstoff ist,
R⁴ Wasserstoff, Kohlenwasserstoff, substituierter Kohlenwasserstoff, oder Silyl ist,
T Wasserstoff, Kohlenwasserstoff oder substituierter Kohlenwasserstoff ist,
L ein Monoolefin oder eine neutrale Lewis-Base ist, wobei das Donor-Atom Stickstoff, Sauerstoff oder Schwefel ist, und
X⁻ ein schwach koordinierendes Anion ist

3. Katalysator-System, umfassend eine Verbindung der Formel XVII: 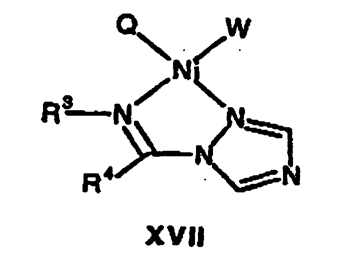 in der
R³ Kohlenwasserstoff oder substituierter Kohlenwasserstoff ist,
R⁴ Wasserstoff, Kohlenwasserstoff, substituierter Kohlenwasserstoff, oder Silyl ist,
Q Kohlenwasserstoff, Chlorid, lodid oder Bromid ist, und
W Kohlenwasserstoff, Chlorid, lodid oder Bromid ist

4. Verfahren zur Herstellung von Polyolefinen, umfassend das Zusammenbringen von einem oder mehreren Olefin-Monomeren der Formel R'CH=CHR", in der R' und R" unabhängig Wasserstoff, Kohlenwasserstoff, Fluoralkyl sind, oder R' und R" unter Bildung eines cyclischen Olefins verbunden sein können, mit einem Katalysator umfassend eine Verbindung der Formel XVII: 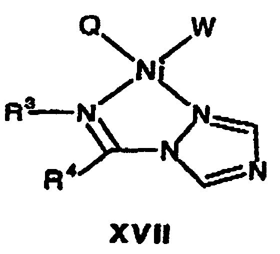 in der
R³ Kohlenwasserstoff oder substituierter Kohlenwasserstoff ist,
R⁴ Wasserstoff, Kohlenwasserstoff, substituierter Kohlenwasserstoff, oder Silyl ist,
Q Kohlenwasserstoff, Chlorid, lodid oder Bromid ist, und
W Kohlenwasserstoff, Chlorid, lodid oder Bromid ist

5. Zusammensetzung, umfassend (i) ein Übergangsmetall M der Gruppen 8-10, (ii) eine oder mehrere Lewis-Säuren und (iii) eine doppelt-kembildende (binucleating) oder mehrfach-kernbildende (mulitnucleating) Verbindung der Formel XVIII: 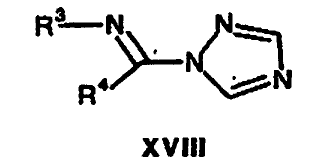 wobei die Lewis-Säure oder ―Säuren an ein oder mehrere Heteroatome gebunden ist/sind, die mit dem Donor-Atom oder -Atomen, das/die an das Übergangsmetall M gebunden ist/sind, π-konjugiert nd.
R³ Kohlenwasserstoff oder substituierter Kohlenwasserstoff ist,
R⁴ Wasserstoff, Kohlenwasserstoff, substituierter Kohlenwasserstoff, oder Silyl ist,

6. Zusammensetzung nach Anspruch 5, wobei das Übergangsmetall M Ni(II) und die Lewis-Säure eine Bor oder Aluminium enthaltende Lewis-Säure ist.

## Revendications

1. Catalyseur comprenant la formule XVI: 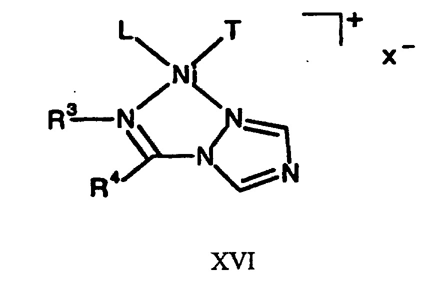 dans laquelle:
R³ est un reste hydrocarbyle ou hydrocarbyle substitué;
R⁴ est un atome d'hydrogène ou un reste hydrocarbyle, hydrocarbyle substitué ou silyle;
T est un atome d'hydrogène ou un reste hydrocarbyle ou hydrocarbyle substitué;
L est une monooléfine ou une base de Lewis neutre dans laquelle l'atome donneur est un atome d'azote, d'oxygène ou de soufre; et
X⁻ est un anion faiblement coordinant.

2. Procédé de préparation de polyoléfines comprenant la mise en contact d'un ou plusieurs monomères oléfiniques ayant la composition R'CH=CHR", où R' et R" sont indépendamment un atome d'hydrogène ou un reste hydrocarbyle ou fluoroalkyle, ou R' et R" peuvent être liés pour former une oléfine cyclique, avec un catalyseur comprenant la formule XVI 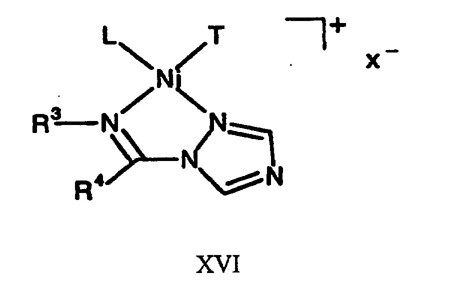 dans laquelle:
R³ est un reste hydrocarbyle ou hydrocarbyle substitué;
R⁴ est un atome d'hydrogène ou un reste hydrocarbyle, hydrocarbyle substitué ou silyle;
T est un atome d'hydrogène ou un reste hydrocarbyle ou hydrocarbyle substitué;
L est une monooléfine ou une base de Lewis neutre dans laquelle l'atome donneur est un atome d'azote, d'oxygène ou de soufre; et
X⁻ est un anion faiblement coordinant.

3. Système catalyseur comprenant un composé de formule XVII: 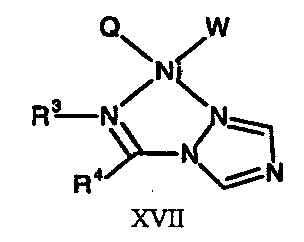 dans laquelle:
R³ est un reste hydrocarbyle ou hydrocarbyle substitué;
R⁴ est un atome d'hydrogène ou un reste hydrocarbyle, hydrocarbyle substitué ou silyle;
Q est un reste hydrocarbyle, chlorure, iodure ou bromure;
W est un reste hydrocarbyle, chlorure, iodure ou bromure.

4. Procédé de préparation de polyoléfines comprenant la mise en contact d'un ou plusieurs monomères oléfiniques de formule R'CH=CHR", dans laquelle R' et R" sont indépendamment un atome d'hydrogène ou un reste hydrocarbyle ou fluoroalkyle, ou R' et R" peuvent être liés pour former une oléfine cyclique, avec un système catalyseur comprenant un composé de formule XVII 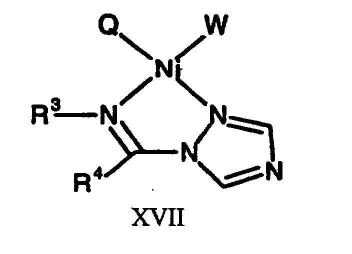 dans laquelle:
R³ est un reste hydrocarbyle ou hydrocarbyle substitué;
R⁴ est un atome d'hydrogène ou un reste hydrocarbyle, hydrocarbyle substitué ou silyle;
Q est un reste hydrocarbyle, chlorure, iodure ou bromure; et
W est un reste hydrocarbyle, chlorure, iodure ou bromure.

5. Composition comprenant (i) un métal de transition M des groupes 8-10, (ii) un ou plusieurs acides de Lewis, et (iii) un composé binucléant ou multinucléant de formule XVIII 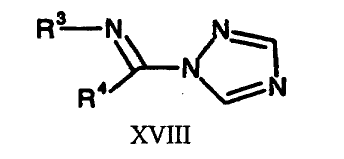 dans laquelle l'acide ou les acides de Lewis sont liés à un ou plusieurs hétéroatomes qui sont conjugués π à l'atome ou aux atomes donneurs liés au métal de transition M;
R³ est un reste hydrocarbyle ou hydrocarbyle substitué;
R⁴ est un atome d'hydrogène ou un reste hydrocarbyle, hydrocarbyle substitué ou silyle.

6. Composition selon la revendication 5, dans laquelle le métal de transition M est Ni(II), et l'acide de Lewis est un acide de Lewis contenant du bore ou de l'aluminium.
